# EUROPÄISCHE PATENTSCHRIFT

(11) **EP 1 809 211 B1**
(45) Veröffentlichungstag und Bekanntmachung des Hinweises auf die Patenterteilung: **24.02.2010**
(21) Anmeldenummer: 05752589.1
(22) Anmeldetag: 13.06.2005
(51) Int. Cl.: A61F 2/44

(54) **PROTHESE ZUR ÜBERBRÜCKUNG EINES WIRBELKÖRPERS**
PROSTHESIS FOR BINDING A VERTEBRAL BODY
PROTHESE DE PONTAGE D'UN CORPS VERTEBRAL

(30) Priorität: 23.09.2004 EP 04022671
(43) Veröffentlichungstag der Anmeldung: 25.07.2007
(73) Patentinhaber: Cervitech, Inc., San Diego CA 92121 (US)
(72) Erfinder: KELLER, Arnold, 23863 Kayhude (DE)
(74) Vertreter: Glawe, Delfs, Moll
(86) Internationale Anmeldenummer: PCT/EP2005/006317
(87) Internationale Veröffentlichungsnummer: WO 2006/032311

(56) Entgegenhaltungen:
- EP-A- 1 290 993
- DE-A1- 4 109 941
- US-A- 4 932 975
- US-A- 5 423 816

## Beschreibung

Die Erfindung betrifft eine Prothese nach dem Oberbegriff des Anspruchs 1. Diesem liegt eine bekannte Prothese zugrunde (DE-A-4109941, Fig. 2), die eine obere Anschlußplatte zur Verbindung mit einem oberen Wirbelkörper, eine untere Anschlußplatte zur Verbindung mit einem unteren Wirbelkörper und einen die obere und die untere Anschlußplatte verbindenden Überbrückungsteil zum Überbrücken mindestens eines zwischen dem oberen und unteren Wirbelkörper befindlichen Wirbelkörpers umfaßt, dessen Funktion durch die Prothese ersetzt werden soll. Zwischen dem Überbrückungskörper und den Anschlußplatten ist je ein Gelenk zum Ersatz der Bandscheiben vorgesehen. Die Querschnittsgröße des Überbrückungsteils ist wesentlich geringer als diejenige dieses Wirbelkörpers. Wenn er mehr oder minder vollständig erhalten ist, soll der Überbrückungsteil in ihn eingesetzt werden, so daß er vollständig darin eingebettet ist. Wie dies operativ geschehen könnte, ist unklar. Wenn er lediglich auf der Wirbelbogenseite noch einigermaßen vollständig ist, wird auf seiner Vorderseite eine Ausnehmung geschaffen, in die der Überbrückungsteil eingesetzt wird. Zur festen Verbindung mit dem Wirbelkörper weist der Überbrückungsteil seitlich vorspringende Stege auf, die ein Langloch zur Aufnahme einer Befestigungsschraube enthalten. Die Befestigung der Prothese am Wirbelkörper bestimmt zusätzlich zu den Facettengelenken dessen Stellung zu den benachbarten Wirbelkörpern. Nur wenn die zur Anlage der Befestigungsstege bestimmten Flächen des Wirbelkörpers so bearbeitet sind, daß dieser nach der Verbindung mit den Stegen seine durch die Facettengelenke vorgegebene natürliche Stellung beibehalten kann, besteht Aussicht auf einen beschwerdefreien Sitz der Prothese. Eine solche genaue Bearbeitung ist schwer zu erreichen. Außerdem hat sich gezeigt, daß eine Schraubbefestigung nicht sicher genug ist.

Bei einer weiteren bekannten Wirbelsäulenprothese (US-A-5423816) ist der Überbrückungsteil von einer Schraubenfeder gebildet, deren Zweck darin besteht, aufgrund ihrer Nachgiebigkeit eine Relativbewegung der oberen und unteren Wirbelkörper im Verhältnis zueinander und zum überbrückten Wirbelkörper zu ermöglichen. Die Spiralfeder soll von einer Höhlung innerhalb des zu überbrückenden Wirbelkörpers aufgenommen werden, der im übrigen mit Knochenspänen gefüllt wird, die möglicherweise das Nachwachsen von Knochengewebe innerhalb der Höhlung ermöglichen. Jedoch ist eine feste Verbindung zwischen den Windungen der Schraubenfeder und dem Knochengewebe wegen deren ständiger Relativbewegung nicht möglich. Die Windungen der Schraubenfeder bilden daher keine Befestigungseinrichtung in bezug auf den überbrückten Wirbelkörper. Im Gegenteil bilden die ständig relativ zu dem überbrückten Wirbelkörper bewegten Windungen der Schraubenfeder einen Anlaß zu fortdauernder Irritation.

Bekannt ist auch eine Wirbelsäulenprothese (EP-A-1417940), bei welcher der Überbrückungsteil in Seitenansicht U-förmig ausgebildet ist, um mit seinen Schenkeln den zu ersetzenden Wirbelkörper unter- und oberseitig zu umfassen. Der Steg liegt auf der Vorderseite des Wirbelkörpers und wird mit diesem verschraubt. Dies setzt eine passende Bearbeitung des wirbelkörper auf seiner Ober-, Unter- und Vorderseite voraus, was insbesondere bei geschädigte Wirbelkörper schwierig sein kann. Ferner sind Wirbelsäulenprothesen bekannt, bei denen der Überbrückungsteil den Wirbelkörper vollständig ersetzt (EP-A-567424, WO 0103614, DE-U-20115281, US-A-5895428). Dies hat den Nachteil, daß eine stützende Verbindung zwischen den verbleibenden Teilen des Wirbels und dem Überbrückungsteil kaum möglich ist.

Bei einer weiteren bekannten Gruppe von Wirbelsäulenprothesen (US-A-4892545, US-A-4636217) wird der Überbrückungsteil starr mit den oberen und unteren Wirbelkörpern verbunden, so daß auch diese starr miteinander verbunden sind. Dadurch wird der überbrückte Wirbelkörper von Kräften frei gehalten und bedarf daher keiner stützenden Verbindung mit dem Überbrückungsteil der Prothese.

Der Erfindung liegt daher die Aufgabe zugrunde, eine Wirbelsäulenprothese der eingangs genannten Art zu schaffen, die auch dann verwendet werden kann, wenn der zu ersetzenden Wirbelkörper vollständig oder zu einem wesentlichen Teil erhalten ist. Sie strebt ferner an, daß eine vergleichsweise einfache Operationstechnik eine gut stützende Verbindung zwischen dem Implantat und dem zu ersetzenden Wirbelkörper verspricht.

Dies gelingt durch die Merkmale des Anspruchs 1. Es ist vergleichsweise leicht, von der Vorderseite her in dem Wirbelkörper eine Ausnehmung zu schaffen, die zu der Gestalt des Überbrückungsteils paßt und diesen im wesentlichen vollständig aufnimmt. Der Überbrückungsteil wird entsprechend schmaler als der Wirbelkörper ausgebildet. Durch den gegenseitigen Formschluß zwischen dem Überbrückungsteil und der Ausnehmung stützen sich der Überbrückungsteil und der Wirbelkörper gegenseitig. Der Überbrückungsteil kann der Ausnehmung auch nicht entweichen, weil seine seitlichen Vorsprünge ihn in der Ausnehmung festhalten.

Zwar ist ein Dübel zum starren Verbinden benachbarter Wirbelkörper bekannt (US-A-2002/0128652) der einen rechteckigen Querschnitt hat und in eine entsprechend angepaßten ventrale Ausnehmung der betroffenen Wirbelkörper eingesetzt wird und mit Einrichtungen ausgerüstet ist, die sein Entweichen aus der Ausnehmung verhindern sollen. Jedoch ist nicht erkennbar, wie sie gestaltet sind.

Die gegenseitige Stützwirkung und die Sicherung durch die seitlichen Vorsprünge des Überbrückungsteils sind um so besser, je genauer die Ausnehmung der Form des Überbrückungsteils angepaßt ist. Dies gelingt am leichtesten dann, wenn die Querschnittsform der Ausnehmung rechteckig oder trapezförmig ist. Dies hat auch den Vorteil, daß die Seitenflächen des Überbrückungsteils groß sind und es dadurch erleichtern, eine Mehrzahl von Verankerungsvorsprüngen unterzubringen. Dies gilt insbesondere dann, wenn diese starr an dem Überbrückungsteil angeordnet sind, beispielsweise in der Form einer Vielzahl von kleinen Spitzen. Diese sind zweckmäßigerweise so gestaltet, daß sie beim Eindrücken des Überbrückungsteils in die Ausnehmung durch elastische oder plastische Nachgiebigkeit des Knochengewebes ihren Weg zu ihrem Verankerungsplatz finden. Dabei hat die sich im Querschnitt nach dorsal verjüngende Form des Überbrückungsteils den weiteren Vorteil, daß beim Einteilen des Überbrückungsteils in die im Querschnitt sich ebenfalls nach dorsal verjüngende Wirbelkörperausnehmung die Vorsprünge in die Knochensubstanz eingesenkt werden. Sie können auch selbstschneidend ausgebildet sein. Eine andere Ausführungsmöglichkeit besteht darin, daß die Vorsprünge als Rauheit sehr klein ausgebildet sind. Dies genügt in der Regel um eine Anfangsfestigkeit der Implantat-Knochen-Verbindung zu schaffen und nach kurzer zeit durch in die Rauhigkeit einwachsendes Knochengewebe eine dauerhafte Verbindung zu ermöglichen. Die Vorsprünge können widerhakenartig ausgeführt sein, um der Bewegung des Im-plantats in die Ausnehmung des Knochens geringen, aber der Entfernung aus der Ausnehmung größeren Widerstand entgegenzusetzen.

Der Überbrückungsteil kann mindestens auf den Seitenflächen Öffnungen oder Poren für die Aufnahme von Knochengewebe aufweisen. Diese können schon vor der Implantation mit Knochenspänen gefüllt werden. Im Laufe der Zeit wächst lebendes Knochengewebe ein, um einen festen Verbund zwischen dem Implantat und den Knochen zu ermöglichen.

Besondere Vorteile hat die Erfindung in der Anwendung bei der Halswirbelsäule.

Die Erfindung wird im folgenden näher unter Bezugnahme auf die Zeichnung erläutert, die vorteilhafte Ausführungsbeispiele veranschaulicht. Es zeigen:
- Fig. 1: einen Längsschnitt eines Ausführungsbeispiels in der Medianebene,
- Fig. 2: eine Frontalansicht desselben Implantats und
- Fig. 3: eine perspektivische Darstellung des Überbrü- ckungsteils.

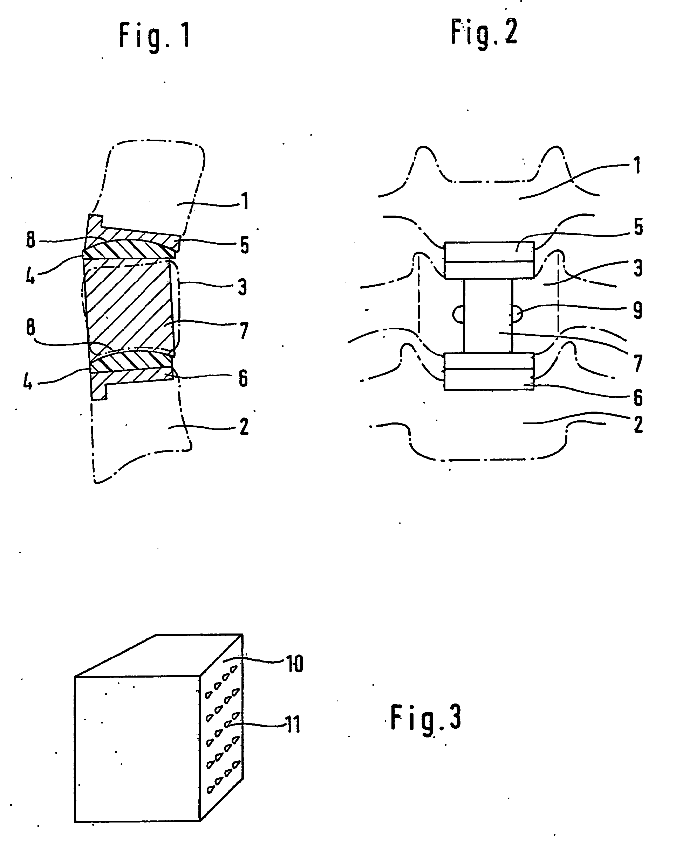

Fig. 2 zeigt einen oberen wirbelkörper 1 und einen unteren Wirbelkörper 2, zwischen denen sich der zu ersetzenden Wirbelkörper 3 befindet. Zwischen dem oberen und dem unteren Wirbelkörper ist eine Wirbelsäulenprothese eingesetzt. Sie umfaßt eine obere Anschlußplatte 5, die mit dem oberen Wirbelkörper 1 verbunden ist, eine untere Anschlußplatte 6, die mit dem unteren Wirbelkörper 2 verbunden ist, und einen Überbrückungsteil 7, der die Platten 5 und 6 verbindet. Zwischen den Anschlußplatten 5 und 6 und dem Überbrückungsteil 7 befindet sich jeweils ein Gelenk (beispielsweise gemäß EP-A-560140) mit einer Gelenkfläche 8. Diese wird im oberen Gelenk einerseits von der Unterseite der Anschlußplatte 5 und andererseits von einem Gelenkteil 4 gebildet, der mit dem Überbrückungsteil 7 in nicht dargestellter Weise verbunden ist. Im unteren Gelenk wird sie einerseits von der Unterseite des Überbrückungsteils 7 und andererseits von dem Gelenkteil 4 gebildet, der mit der unteren Anschlußplatte 6 in nicht dargestellter Weise verbunden ist. Statt eines Gelenks mit sphärischer Gelenkfläche kann auch ein anderer Gelenktyp verwendet werden, beispielsweise ein solcher mit flexiblem Kissen (DE-U-20115281) oder mit Biegefeder (DE-A-4109941). Falls der obere Wirbelkörper 1 und der untere Wirbelkörper 2 starr verbunden werden sollen, können die Gelenke auch gänzlich entfallen. Eine solche Prothese stellt aber keinen Teil der vorliegenden Erfindung dar. Schließlich ist es möglich, nur ein Gelenk zwischen der oberen Anschlußplatte 5 und dem Überbrückungsteil 7 oder zwischen der unteren Anschlußplatte 6 und dem Überbrückungsteil 7 zu verwenden.

Während die Anschlußplatten 5 und 6 eine übliche Größe haben, die im Interesse geringer Druckkräfte zwischen den Anschlußplatten und den zugehörigen Wirbelkörpern bemessen ist, hat der Überbrückungsteil 7 eine Breite, die geringer ist als diejenige des zugehörigen Wirbelkörpers 3, nämlich so gering, daß der Überbrückungsteil in eine von der Vorderseite in den betreffenden Wirbelkörper 3 eingearbeitete Ausnehmung eingesetzt werden kann und die daneben verbleibende Knochensubstanz zur sicheren Verankerung des Überbrückungsteils in der Ausnehmung ausreicht.

Zumindest ein Teil der Ausnehmung hat eine Gestalt, die möglichst genau mit der Gestalt des Überbrückungsteils 7 übereinstimmt. Dadurch wird eine im wesentlichen spielfreie Anlage der Implantatoberfläche an der künstlich geschaffenen Oberfläche des Knochens möglich. Zum einen ergibt dies eine gute gegenseitige Abstützung. Zum anderen ergibt sich daraus die Möglichkeit einer durch Knochenwachstum entstehenden stabilen Verbindung zwischen dem Knochen und dem Implantat. Und schließlich gibt dies Sicherheit dafür, daß die am Implantat vorgesehenen Verankerungsvorsprünge 9 mit im wesentlichen ihrer vollen Länge in das Knochengewebe eingreifen, um die Verankerungskräfte übertragen zu können.

Das Ziel der Formübereinstimmung zwischen dem Überbrückungsteil und der im Knochen geschaffenen Ausnehmung erreicht man am leichtesten mit einer Trapezform des Querschnitts des Überbrückungsteils, wie sie in Fig. 3 angedeutet ist. Es können auch andere Querschnittsformen verwendet werden, vorzugsweise aber solche, die sich von vorne nach hinten verjüngen, damit beim Einsetzen des Überbrückungsteils in die Ausnehmung die erwähnte spielfreie Anlage der Seitenflächen an den Resektionsflächen des Knochens zustandekommt.

Das Merkmal, daß der Überbrückungsteil eine nach hinten schmaler werdende, trapezförmige Querschnittsform hat, soll Schutz genießen.

Die Verankerungsvorsprünge sind starr an den Seitenflächen 10 des Überbrückungsteils 7 angeordnet. Gemäß Fig. 3 haben sie die Form einer Vielzahl von kleinen, spitzen Erhebungen 11, die beim Einschieben des Implantats in die Ausnehmung des Wirbels sich in die Knochenoberfläche eindrücken. In einer nicht dargestellten alternativen Ausführung sind sie größer und klingenförmig ausgebildet, wobei die Klingenebene in Einschubrichtung verläuft, so daß sie selbstschneidend in die Knochensubstanz eindringen. Eine wieder andere Ausführungsform verwendet Mikrovorsprünge in Gestalt einer Rauhigkeit, welche die gesamte Seitenfläche 10 oder einen wesentlichen Teil derselben überzieht. In jedem Fall können zusätzlich zu den Verankerungsvorsprüngen Öffnungen 12 oder Poren vorgesehen sein, in denen hineinwachsende Knochensubstanz sich verankern kann. Um diesen Prozeß zu beschleunigen, können die Öffnungen vorab mit Knochenspänen gefüllt werden. Auch eine Beschichtung des Implantats mit osteokonduktiver oder osteoinduktiver Substanz ist möglich.

## Patentansprüche

1. Prothese zum Teilersatz eines Wirbelkörpers mit einer oberen Anschlußplatte (5) zur Verbindung mit einem oberen Wirbelkörper (1), einer unteren Anschlußplatte (6) zur Verbindung mit einem unteren Wirbelkörper (2) und einem die obere und die untere Anschlußplatte (5, 6) unter Einschluß mindestens eines Gelenks (8) verbindenden Überbrückungsteil (7), der zum Überbrücken mindestens eines zwischen dem oberen und dem unteren Wirbelkörper (1, 2) befindlichen und teilweise zu ersetzenden Wirbelkörpers (3) ausgebildet ist und mit seitlich vorragenden, starren Verankerungsvorsprüngen (9, 11) zur Befestigung am Wirbelkörper (3) ausgerüstet ist, **dadurch gekennzeichnet, daß** die Querschnittsform des Überbrückungsteils (7) rechteckig oder trapezförmig und nach dorsal schmaler werdend ist.

2. Wirbelsäulenprothese nach Anspruch 1, **dadurch gekennzeichnet, daß** die Verankerungsvorsprünge als Rauheit ausgebildet sind.

3. Wirbelsäulenprothese nach Anspruch 1, **dadurch gekennzeichnet, daß** der Überbrückungsteil (7) Öffnungen (12) oder Poren für die Aufnahme von Knochengewebe aufweist.

4. Wirbelsäulenprothese nach Anspruchs 3, **dadurch gekennzeichnet, daß** sie mit einer Füllung von Knochenmaterial oder Knochenersatzmaterial versehen ist.

## Claims

1. Prosthesis for partial replacement of a vertebral body, with an upper contact plate (5) for connection to an upper vertebral body (1), a lower contact plate (6) for connection to a, lower vertebral body (2), and a bridging part (7) which connects the upper and lower contact plates (5, 6) to one another, with inclusion of at least one hinge (8), and is configured for bridging at least one vertebral body (3) which is located between the upper and lower vertebral bodies (1, 2) and is to be partially replaced, said bridging part (7) being equipped with rigid anchoring projections (9, 11) which protrude laterally for securing it on the vertebral body (3), **characterized in that** the cross-sectional shape of the bridging part (7) is rectangular or trapezoid and narrows in the dorsal direction.

2. Spinal column prosthesis according to Claim 1, **characterized in that** the anchoring projections are in the form of a surface roughness.

3. Spinal column prosthesis according to Claim 1, **characterized in that** the bridging part (7) has openings (12) or pores for receiving bone tissue.

4. Spinal column prosthesis according to Claim 3, **characterized in that** it is provided with a filling of bone material or bone replacement material.

## Revendications

1. Prothèse pour le remplacement partiel d'un corps vertébral comportant une plaque de liaison supérieure (5) pour la jonction avec un corps vertébral supérieur (1), une plaque de liaison inférieure (6) pour la jonction avec un corps vertébral inférieur (2) et une pièce de pontage (7) reliant la plaque de liaison supérieure et la plaque de liaison inférieure (5, 6) en incluant au moins une articulation (8), laquelle pièce est destinée à ponter au moins un corps vertébral (3) à remplacer partiellement et se trouvant entre le corps vertébral supérieur et le corps vertébral inférieur (1, 2) et est munie de saillies d'ancrage (9, 11) rigides saillantes latéralement pour la fixation sur le corps vertébral (3), **caractérisée en ce que** la forme de la section transversale de la pièce de pontage (7) est rectangulaire ou trapézoïdale et se rétrécit vers la face dorsale.

2. Prothèse de colonne vertébrale selon la revendication 1, **caractérisée en ce que** les saillies d'ancrage sont réalisées sous la forme d'une rugosité.

3. Prothèse de colonne vertébrale selon la revendication 1, **caractérisée en ce que** la pièce de pontage (7) présente des ouvertures (12) ou des pores destinés à recevoir du tissu osseux.

4. Prothèse de colonne vertébrale selon la revendication 3, **caractérisée en ce qu'**elle est pourvue d'un remplissage de matière osseuse ou de matière de substitution osseuse.
